# EUROPEAN PATENT SPECIFICATION

(11) **EP 4 574 784 B1**
(45) Date of publication and mention of the grant of the patent: **20.05.2026**
(21) Application number: 24156299.0
(22) Date of filing: 07.02.2024
(51) Int. Cl.: C03C 1/00, C03C 3/19, C03C 4/00

(54) **A METHOD FOR PRODUCTING BIOACTIVE BORATE GLASSES**
VERFAHREN ZUR HERSTELLUNG VON BIOAKTIVEN BORATGLÄSERN
PROCÉDÉ DE PRODUCTION DE VERRES BIOACTIFS AU BORATE

(30) Priority: 21.12.2023 PL 44720123
(43) Date of publication of application: 25.06.2025
(73) Proprietor: Akademia Gorniczo-Hutnicza im. Stanislawa Staszica w Krakowie, 30-059 Krakow (PL)
(72) Inventor: Dziadek, Michal, 32-083 Balice (PL); Cholewa-Kowalska, Katarzyna, 30-821 Krakow (PL); Salagierski, Szymon, 30-150 Krakow (PL); Gackowska, Patrycja, 31-621 Krakow (PL)
(74) Representative: Pietruszynska, Elzbieta

(56) References cited:
- WO-A1-2015/188252
- KERMANI FARZAD ET AL: "Modified Sol-Gel Synthesis of Mesoporous Borate Bioactive Glasses for Potential Use in Wound Healing", BIOENGINEERING, vol. 9, no. 9, 5 September 2022 (2022-09-05), pages 442, XP093182296, ISSN: 2306-5354, DOI: 10.3390/bioengineering9090442

## Description

The invention relates to a method for producing bioactive borate glasses. The invention relates to the field of producing bioactive borate glasses with different chemical composition, in the form of powders, using a low-temperature technique process for obtaining inorganic glasses - wet chemical synthesis (sol-gel).

Bioactive glasses are used with great success in medicine, primarily in the field of orthopedics and dentistry. The first chemical composition of glass showing bioactivity, named as 45S5 Bioglass®, was discovered by L.L. Hench in 1969. In this glass, the basic glass-forming component is silicon oxide (silicate glass). The other ingredients are sodium oxide, calcium oxide and phosphorus oxide. Glass is produced using traditional melting techniques. Commercially available products, based on the 45S5 composition, are used, among others, to fill bone defects in periodontium, mandible and maxilla (PerioGlas^{®}), to fill other defects in the skeletal system (NovaBone^{®}, Glassbone^{®}), and in the treatment of tooth hypersensitivity (NovaMin^{®}). Other glass used in the clinic, also from the system SiO₂-Na₂O-CaO-P₂O₅, obtained by melting technique, has composition named as S53P4. It is used for bone fillings of various origins (BonAlive^{®}). The main feature of bioactive glasses, determining their use as alloplastic implants, is the ability to create a permanent bond with tissues (bioactivity), both bone and soft tissues. This process is carried out by modifying a surface layer of glass in contact with body tissues/fluids. It involves migration of modifying ions (sodium, calcium) from the glass structure to the tissue surroundings (depletion of the surface layer of glass), resulting in the formation of a layer of highly polymerized silica gel. It is the nucleation site of biomimetic carbonate hydroxyapatite (CHA), which is directly responsible for bonding the material to tissues. These processes can be observed in vitro in contact of bioactive glass with various types of solutions/buffers (e.g. simulated blood plasma - SBF, TRIS-based buffers, phosphate buffers). This phenomenon is often used for the initial assessment of the bioactivity of glasses. Under in vivo conditions, the bond formation process additionally includes the step of adsorption of collagen and non-collagen proteins and adhesion of bone-forming cells (osteoconductive properties). Bioactive glasses with appropriately designed chemical composition also stimulate regenerative processes of bone tissue (osteoinductive properties). Degradation products of bioactive silicate glasses activate a number of genes responsible for osteogenesis, including differentiation of bone progenitor cells, including production of extracellular matrix proteins and its mineralization. Bioactive glasses with osteoinductive properties include 45S5 and glasses produced using the sol-gel method (e.g. 58S). Bioactive silicate glasses also have an angiogenesis-stimulating effect, so they can be used in regeneration of soft tissues. Glasses with confirmed angiogenic properties include 45S5 and 13-93. Bioactive silicate glasses, especially those obtained using the sol-gel method, may have hemostatic effects (WO2014150224A1). Glasses obtained using the wet chemical synthesis technique (sol-gel) are characterized by the presence of porosity and a much higher specific surface area compared to glasses produced using the traditional melting method. This causes that the rate and degree of release of degradation products from their structure to be higher, and therefore these glasses are characterized by improved bioactivity and higher biological activity compared to fused glasses with the same chemical composition. The faster formation of the CHA layer on the surface of glasses obtained using the sol-gel technique is additionally promoted by the presence of Si-OH silanol groups. An important advantage of using the sol-gel method to produce silicate glasses is the ability to eliminate fluxes (alkali metal oxides, primarily sodium oxide) from the glass composition, which are necessary in the traditional melting process.

Another oxide capable of forming a glass-like bond is boron oxide (borate glasses). The recent increase in interest in bioactive borate glasses is primarily due to their greater solubility/reactivity in the biological environment compared to silicate glasses. This makes them suitable for use in the treatment/regeneration of soft tissues, including skin. The only commercially available borate glass for medical applications is glass from the system B₂O₃-Na₂O-K₂O-MgO-CaO-P₂O₅, with a composition named as 13-93B3. Glass in the form of glass fibers produced by melting technique is used as a dressing for various wounds, including difficult-to-heal ones (Mirragen^{®}). Bioactive borate glasses exhibit a different mechanism of conversion to CHA compared to silicate glasses. The greater solubility of borate glasses enables ion exchange not only on the surface of the material, as is case of silicate glasses, but throughout its entire volume. This leads to complete conversion of borate glass into hydroxyapatite, so they can be a substrate for the production of biomimetic CHA. Bioactive borate glasses, apart from stimulating tissue regeneration properties (e.g. bone tissue, skin), also have angiogenic and antibacterial properties. Borate glasses, including bioactive ones, are obtained mainly by the traditional melting method. The limited interest in the sol-gel technique in this area results primarily from the higher degree of complexity of the chemistry of sol-gel processes in borate systems compared to silicate systems, which results from the variable coordination of boron in boron-oxygen units (possible coordination 3 and 4). [10.1016/C2009-0-22386-5]. Gel formation in case of borate systems is difficult due to the lower ability of boron-oxygen units to create spatial, sufficiently polymerized structure. [10.1021/acs.chemmater.5b01697]. Alkali metal oxides (e.g. Na₂O) or alkaline earth metal oxides (CaO) are introduced into borate glasses synthesized using the sol-gel technique, most often using alkoxides (ethoxides or methoxides). These substances undergo hydrolysis very easily/quickly, producing insoluble oxides or hydroxides. For this reason, synthesis using them must be carried out in a protective (anhydrous) atmosphere. [10.1021/acs.chemmater.5b01697; 10.1007/s10853-017-0968-y, WO2015188252A1], which significantly complicates the entire process and limits its use in the production of borate glasses, especially on a larger (industrial) scale. The mentioned alkoxides have significantly limited solubility, therefore the introduction of an appropriate amount of the compound (metal oxide) generates a large volume of the entire synthesis, and thus causes its low efficiency. However, the use of the sol-gel method in case of borate glasses for medical applications has several advantages. As in case of silicate glasses, it allows obtaining porous glasses with a significant surface area, increasing the reactivity of the materials in the biological environment. The second advantage of the sol-gel method is the possibility of obtaining borate glasses with a high calcium oxide content, which is not possible with the melting technique due to the high tendency of glasses to crystallize. [10.1039/D0MA00360C; 10.1016/j.jnoncrysol.2023.122406].

In the publications No. WO2020198859A1, WO2015188252A1, US10624994B2 relating to borate glasses produced by the sol-gel method, the described process of their production consists of the following steps: (i) mixing precursors, possibly in the presence of a solvent, to obtain a solution and then a sol, (ii) aging sol to obtain gel, (iii) aging/drying gel, (iv) thermal treatment (calcination), (v) possible grinding process to obtain powders with a desired grain fraction. The gelation process may occur (I) already during mixing the precursor or (II) during aging sol without solvent evaporation or (III) during aging sol as a result of solvent evaporation (drying). The gel drying step in the described solutions is a process of removing the liquid phase by conventional evaporation, possibly supported by increased temperature.

In the publications No. WO2020198859A1 and WO2015188252A1, in the sol-gel process it may be necessary to adjust pH of the solution at a level from about 10.5 to about 14.0 or from about 11 to about 13.5 and to use a protective atmosphere during the synthesis (use of a glove box with nitrogen atmosphere).

The publication No. US10624994B2, apart from indicating the standard sol-gel method, also mentions a modification of this process. It involves sonicating sol and using microwave radiation to precipitate a sediment, and then drying and thermally treating the obtained sediment at a temperature of 600-800°C in order to obtain bioactive glass in the form of powder. This publication focuses primarily on the production of pastes based on bioactive borate glasses, used to rebuild damaged enamel and dentin. The publication does not describe/claim parameters of the sol-gel synthesis process, as is case of publications WO2020198859A1 and WO2015188252A1.

In the publication 10.3390/bioengineering9090442, freeze-drying was used as a method of drying gel that had previously been aged. The publication describes a method for producing silver-doped bioactive borate glass 13-93B3 by the sol-gel method. However, the technique used did not lead to obtaining 100% amorphous material. Thermal treatment of the material obtained after freeze-drying led to obtaining a glass-crystalline material in which the crystalline phase was calcium borate.

The publication WO2009013497A1 mentions freeze-drying as one of the methods for drying gel in production of bioactive silicate glasses by the sol-gel method.

The publication WO2017176724A1A mentions freeze-drying as one of the methods for removing organic residues and impurities from bioactive glasses and glass-ceramics produced by the sol-gel method. In this publication, gel or gel subjected to other additional processing is subjected to the freeze-drying process.

In methods known from the prior art, the steps of sol aging and wet gel aging and drying are long-term processes. Furthermore, the duration of steps of sol aging and wet gel aging and drying is difficult to control. The object of the present invention is to provide a method for producing bioactive borate glasses which is free from these drawbacks.

The invention relates to a method for producing bioactive borate glasses, comprising the following steps:
a) mixing of precursors of B₂O₃, CaO, Na₂O and P₂O₅ in a solvent for 12-48 hours, in a temperature from 20 to 40°C, to obtain a sol;
b) freezing the sol obtained in step a) in a temperature from -10 to -196 °C, and then freeze-drying to obtain an aerogel;
c) grinding the aerogel obtained in step b) and sieving through a sieve with a mesh size of 40-100 µm;
d) heating the aerogel sieved in step c) according to the following temperature treatment scheme:
   - heating the aerogel at a speed ranging from 1,5 to 3 °C/min up to a temperature ranging from 100 to 150 °C;
   - maintaining the aerogel in a temperature ranging from 100 to 150 °C, over time from 24 to 72 hours;
   - heating the aerogel at a speed ranging from 1,5 to 3 °C/min, up to a temperature ranging from 400 to about 600 °C;
   - maintaining the aerogel in a temperature ranging from 400 to about 600 °C, over time from 1,5 to 6 hours;
   - free cooling of the furnace.

Preferably, the precursor of B₂O₃ is boric acid, trimethyl borate, triethyl borate, tripropyl borate, tributyl borate, tri-tert-butyl borate or trimethoxyboroxin.

Preferably, the precursor of CaO is calcium chloride, calcium nitrate, calcium acetate, calcium lactate, calcium citrate, calcium methoxide, calcium ethoxide or calcium methoxyethoxide. Preferably, the precursor of Na₂O is sodium chloride, sodium nitrate, sodium acetate, sodium lactate, sodium citrate, sodium methoxide or sodium ethoxide.

Preferably, the precursor of P₂O₅ is trimethyl phosphate, triethyl phosphate, tripropyl phosphate, tributyl phosphate, dibutyl phosphate, phosphoryl trichloride and phosphoric acid. Preferably, in step a), to the mixture of the precursors of B₂O₃, CaO, Na₂O and P₂O₅ in a solvent, precursors of K₂O, Li₂O, MgO, SrO, TiO₂, MnO₂, Fe₂O₃, CoO, NiO, CuO, ZnO, Ag₂O, Au₂O₃, Al₂O₃ or Ga₂O₃, or combinations thereof, are added.

Preferably, the precursors of K₂O, Li₂O, MgO, SrO, TiO₂, MnO₂, Fe₂O₃, CoO, NiO, CuO, ZnO, Ag₂O, Au₂O₃, Al₂O₃ or Ga₂O₃ are inorganic salts of specific elements, selected from the group comprising chlorides, nitrates, acetates, lactates, citrates and alcoholates.

Preferably, the solvent is an organic solvent whose melting point is ≥ 0°C.

Preferably, the solvent is 1,4-dioxane, dimethyl carbonate, tert-butanol or an aqueous solution of these compounds.

Preferably, the volume ratio of the precursor of boron to the solvent is from 1:1 to 1:3.

Preferably, the product obtained in step d) is subjected to grinding.

The method according to the invention allows obtaining glasses with the content of individual oxides: B₂O₃ - 35-90 wt.%, CaO - 10-50 wt.%, Na₂O - 0-40 wt.% and P₂O₅ - 0-15 wt.%. Glasses can be modified with the above-mentioned oxides K₂O, Li₂O, MgO, SrO, TiO₂, MnO₂, Fe₂O₃, CoO, NiO, CuO, ZnO, Ag₂O, Au₂O₃, Al₂O₃, Ga₂O₃ in an amount of 0-15 wt.%. The amounts of precursors of oxides added in the method according to the invention result directly from the assumed chemical composition of glass.

Unlike the solutions known in the prior art, the method according to the present invention completely eliminates the stages of obtaining gel, sol aging and wet gel aging and drying, which are entirely replaced by the process of freezing/freeze-drying of the sol. Complete elimination of long stages of sol aging and wet gel aging and drying, and replacing them with the freezing/freeze-drying of sol significantly shortens the synthesis time. Thus, the processes of sol aging and wet gel aging and drying, which are difficult to control (depending on, among others, a composition of the produced glasses, precursors used, synthesis conditions - e.g. temperature, humidity) have been eliminated, which allows for precise determination of the duration of the entire synthesis, ensuring its high repeatability and therefore allows for easy scaling. The proposed solution does not require the use of special synthesis conditions (protective atmosphere, pH of the solution/sol).

The invention is illustrated by the following examples and drawings.
Fig. 1a and 1b show the Scheme of sol-gel synthesis and aerogel temperature treatment for glasses produced according to example 1.
Fig. 2a-2b show XRD diffractograms and FTIR absorption spectra for glasses produced according to examples 1, 2 and 3.
Fig. 3a-3d show FTIR absorption spectra for some glasses produced according to examples 1 and 3 before and after 3-, 7- and 14-days incubation in simulated plasma (SBF).

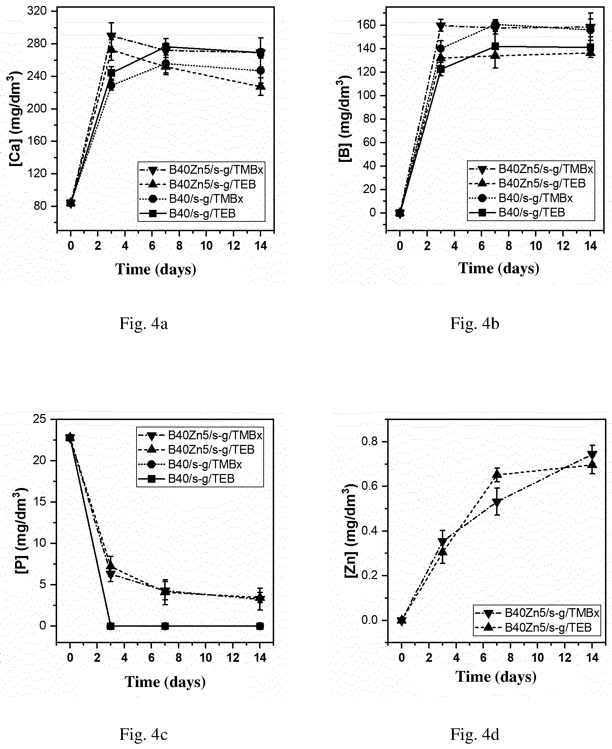

Fig. 4a-4d show changes in the concentration of boron, calcium, phosphorus and zinc in simulated plasma (SBF) during incubation of glasses produced according to examples 1 and 3.

### Example 1: A method for producing glass by the sol-gel method, having the composition: 41.8 wt.% B₂O₃, 45.4 wt.% CaO, 12.8 wt.% P₂O₅ (40 mol% B₂O₃, 54 mol% CaO, 6 mol% P₂O₅), using various precursors of B₂O₃.

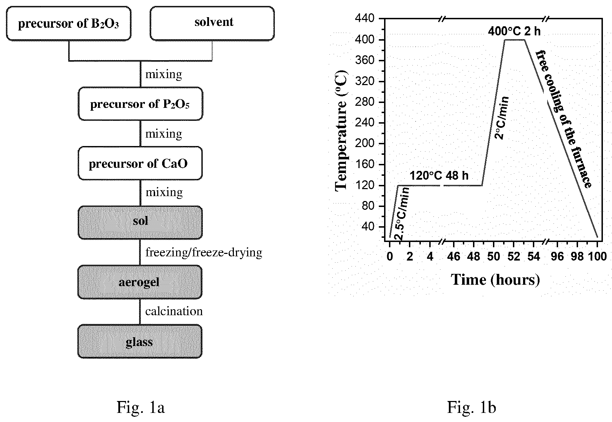

The precursor of B₂O₃ (trimethyl borate or triethyl borate or tributyl borate or trimethoxyboroxin) is mixed with the solvent in the form of 1,4-dioxane in the volume ratio of 1:1.5 for 15 minutes. Then, the precursor of P₂O₅ in the form of triethyl phosphate is added and the solution is mixed for 30 minutes. In the next step, the precursor of CaO in the form of an aqueous solution of calcium chloride dihydrate is added and the mixing process is continued for 24 hours. The amounts of precursors of individual oxides added to the synthesis are presented in Table 1. The sol obtained as a result of mixing is transferred to a Petri dish, frozen at a temperature of approximately -20 °C for 24 hours, and then freeze-dried for 48 hours (a collector temperature of -103 °C , vacuum 0.12 mbar). The obtained aerogel is crushed and sieved through a 100 µm sieve, and then subjected to thermal treatment according to the following scheme: heating at the rate of 2.5 °C/min to a temperature of 120 °C; maintaining at 120 °C for 48 hours; heating at the rate of 2 °C/min to a temperature of 400 °C; maintaining at 400 °C for 2 hours; free cooling of the furnace. The obtained powder is then crushed and sieved through a 100 µm sieve. The temperature treatment scheme of the aerogel is shown in Fig. 1a and 1b. Further in the description, glasses are defined in accordance with Table 1.

**Table 1. Definitions of glasses prepared according to example 1 with amounts of precursors of individual oxides used in the synthesis.**

| **Definitions of glass** | **Used precursor of B₂O₃** | **Amount of precursors of individual oxides used in the synthesis (g)** | | |
|---|---|---|---|---|
| | | **B₂O₃** | **CaO** | **P₂O₅** |
| B40/s-g/TMB | trimethyl borate | 18.64 | 17.63 | 4.86 |
| B40/s-g/TEB | triethyl borate | 17.16 | 11.66 | 3.22 |
| B40/s-g/TBB | tributyl borate | 17.06 | 7.36 | 2.03 |
| B40/s-g/TMBx | trimethoxyboroxin | 23.90 | 39.44 | 10.86 |

### Example 2: A method for producing glass by the traditional melting method, having the composition shown in example 1.

Glass with the composition shown in example 1 was produced by the traditional melting method in order to compare its properties with those of the produced glass using the proposed sol-gel technique. Precursors of B₂O₃, CaO and P₂O₅ in the form of H₃BO₃, CaCO₃, P₂O₅, respectively, were homogenized and then placed in a PtRh₅ crucible and melted at 1200 °C for 30 minutes. The resulting alloy was then quickly cooled by thermal shock (cooling medium: water). Glass was immediately drained on a filter paper and then dried at 120 °C for 48 hours. Then, they were crushed and sieved through a 100 µm sieve. The melting method was used for comparative purposes in relation to glasses produced using the sol-gel technique and is not the subject of this application. Further in the description, glass was named as B40/m-q.

### Example 3: A method for producing glass by the sol-gel method, having the composition shown in example 1, wherein calcium oxide was replaced by zinc oxide or copper oxide or cobalt oxide in an amount of 5 mol% (40 mol% B₂O₃, 49 mol% CaO, 6 mol% P₂O₅, 5 mol% MeO, wherein Me is Zn or Cu or Co), using various precursors of B₂O₃.

The precursor of B₂O₃ (triethyl borate or trimethoxyboroxin) is mixed with the solvent in the form of 1,4-dioxane in the volume ratio of 1:1.5 for 15 minutes. Then, the precursor of P₂O₅ in the form of triethyl phosphate is added and the solution is mixed for 30 minutes. In the next step, the precursor of CaO in the form of an aqueous solution of calcium chloride dihydrate is added and the mixing process is continued for 30 min. Then, the precursor of ZnO or CuO or CoO in the form of an aqueous solution of anhydrous zinc chloride or copper chloride dihydrate or cobalt chloride hexahydrate respectively is added and the mixing process is continued for 24 hours. The amounts of precursors of individual oxides added to the synthesis are presented in Table 2. The sol is obtained, for which the further procedure is identical to that described in example 1. Further in the description, glasses are defined in accordance with Table 2.

**Table 2. Definitions of glasses prepared according to example 3 with amounts of precursors of individual oxides used in the synthesis.**

| **Definitions of glass** | **Used precursor of B₂O₃** | **Modification of MeO** | **Amount of precursors of individual oxides used in the synthesis (g)** | | | |
|---|---|---|---|---|---|---|
| | | | **B₂O₃** | **CaO** | **P₂O₅** | **MeO** |
| B40Cu5/s-g/TEB | triethyl borate | CuO | 17.16 | 10.59 | 3.22 | 1.25 |
| B40Cu5/s-g/TMBx | trimethoxyboroxin | CuO | 23.90 | 35.79 | 10.86 | 4.23 |
| B40Co5/s-g/TEB | triethyl borate | CoO | 17.16 | 10.59 | 3.22 | 1.75 |
| B40Co5/s-g/TMBx | trimethoxyboroxin | CoO | 23.90 | 35.79 | 10.86 | 5.91 |
| B40Zn5/s-g/TEB | triethyl borate | ZnO | 17.16 | 10.59 | 3.22 | 1.01 |
| B40Zn5/s-g/TMBx | trimethoxyboroxin | ZnO | 23.90 | 35.79 | 10.86 | 3.42 |

### Example 4: Structure of glasses produced according to examples 1, 2 and 3.

XRD diffraction pattern were recorded using a X'Pert Pro (PANalytical B.V., Netherlands) diffractometer in the range of angles 2θ of 10-90° with the step of 0,0170°, using monochromatic radiation CuK_{α}, with a lamp operating parameters of 40 kV of voltage and 40 mA of current. For identification of crystalline phases, HighScore Plus 3.0.4 (PANalytical B.V., Netherlands) software and diffraction databases ICDD PDF-2 was used. FTIR spectra were recorded using Bruker Vertex 70V (Bruker Optik GmbH, Germany) spectrometer in the transmission technique with KBr pellet, in the range of 400-4000 cm⁻¹, with resolution of 4 cm⁻¹, using 124 scans for each sample. Baseline correction and vector normalization were performed for all spectra using OPUS 7.2 (Bruker Optik GmbH, Germany) software.

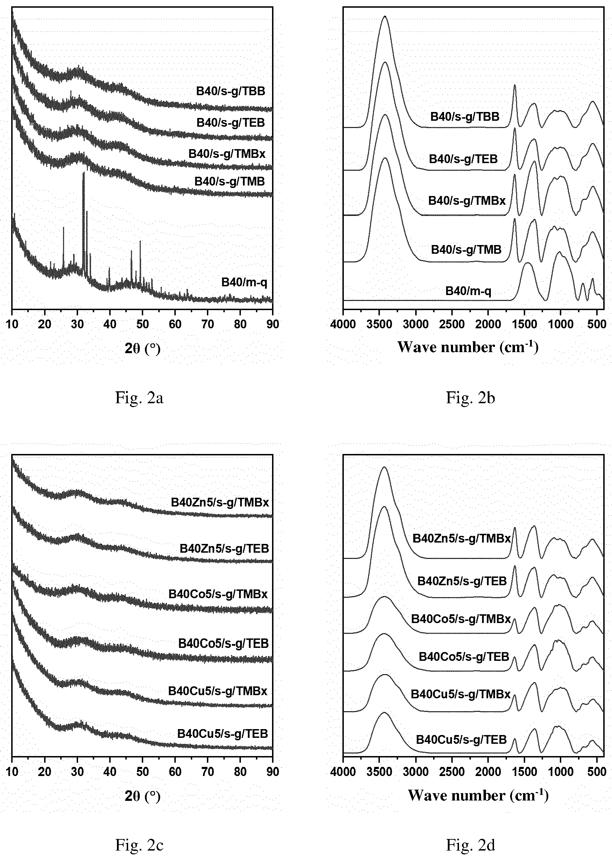

In Figs. 2a-2d. XRD diffractograms and FTIR absorption spectra of glasses prepared according to examples 1, 2 and 3 are shown. Diffractograms of all glasses prepared by the sol-gel method, both unmodified (B40/s-g/TMB, B40/s-g/TEB, B40/s-g/TBB, B40/s-g/TMBx), as well as modified CuO, CoO and ZnO (B40Cu5/s-g/TEB, B40Cu5/s-g/TMBx, B40Co5/s-g/TEB, B40Co5/s-g/TMBx, B40Zn5/s-g/TEB, B40Zn5/s-g/TMBx) indicate their amorphous nature. In glass produced by the melting method (B40/m-q) with the composition identical to that obtained by the sol-gel method, apart from the amorphous phase, crystallization of the phosphate-borate-calcium phase (ICCD #01-080-0537) is visible. The FTIR spectra of glasses produced by the sol-gel and melting methods show bands characteristic for the boron-oxygen bond of glasses. These bands are located in three areas: 800-1250 cm⁻¹ (vibrations stretching B-O in BO₄ tetrahedrons), 1250-1580 cm⁻¹ (vibrations stretching B-O in BO₃ trigonal units), ~690 cm⁻¹ (vibrations bending B-O-B in BO₃ trigonal units). In case of glasses prepared by the sol-gel method, a single band at approximately 560 cm⁻¹ and a band at approximately 1080 cm⁻¹ are visible, characteristic for vibrations, respectively, bending O-P-O and stretching P-O of PO₄³⁻ groups in the morphic structure. For glass produced by melting technique, two sharp bands at 560 and 605 cm⁻¹ and a band at 1020 cm⁻¹ are visible, characteristic for vibrations, respectively, bending O-P-O and stretching P-O of PO₄³⁻ groups in the crystal structure. This is confirmed by results of the XRD analysis regarding the appearance of a crystalline phase in case of glass obtained by melting method. The spectra of glasses prepared using the sol-gel method show a sharp band at 1640 cm⁻¹ and a broad band in the range of 2900-3800 cm⁻¹ corresponding to the vibrations of O-H groups in the water adsorbed in the samples. XRD and FTIR analysis indicate that a type of a precursor of B₂O₃ used in example does not have significant impact on the structure of the boron-oxygen bond of glasses.

### Example 5: In vitro bioactivity of some glasses prepared according to examples 1 and 3 - ability to transform into the phosphate-calcium phase

Ability to transform into the phosphate-calcium phase (mineralization) of some glasses prepared according to examples 1 (B40/s-g/TEB, B40/s-g/TMBx) and 3 (B40Zn5/s-g/TEB, B40Zn5/s-g/TMBx) was assessed by incubation in artificial plasma (SBF, pH 7.4), the composition of which was developed by T. Kokubo. Glass powders were placed in sterile, tightly closed containers containing SBF (3 mg/1 ml) and incubated in 37°C±1°C. After 3, 7 and 14 days of incubation, the powders were filtered on a filter paper and dried at 60°C for 72 hours. Then, FTIR analysis was performed.

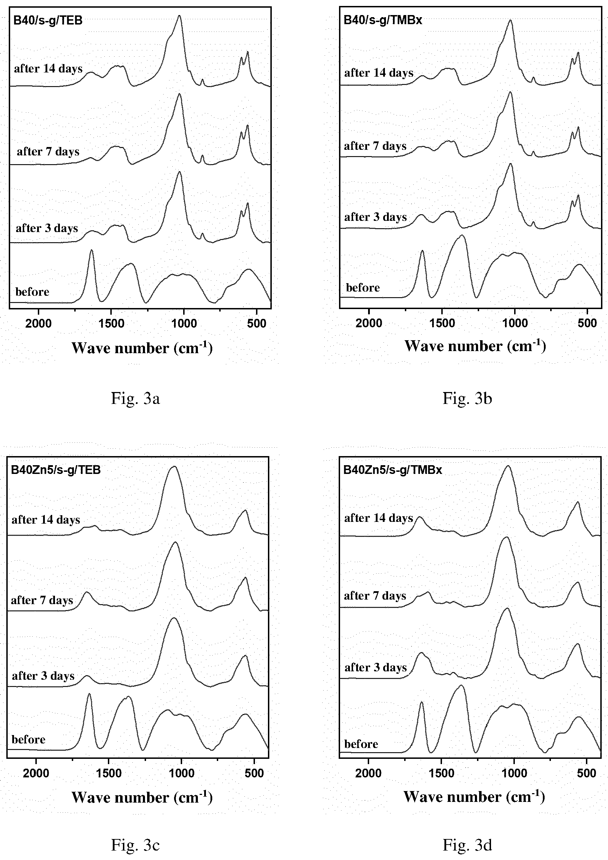

Figures 3a-3d show FTIR absorption spectra of glasses before and after 3-, 7- and 14-day incubation in SBF. After incubation in SBF, on the spectra of both unmodified glasses (B40/s-g/TEB, B40/s-g/TMBx), intense bands appear, characteristic for the crystalline calcium-phosphate phase - carbonate hydroxyapatite: 560 cm⁻¹ and 600 cm⁻¹ (vibrations bending O-P-O of PO₄³⁻ groups), 960 cm⁻¹ (symmetrical vibrations stretching P-O of PO₄³⁻ groups), 1035 cm⁻¹ and 1100 cm⁻¹ (asymmetrical vibrations stretching P-O of PO₄³⁻ groups), 870 cm⁻¹ and 1350-1550 cm⁻¹ (respectively, bending vibrations and asymmetrical stretching vibrations of CO₃²⁻ groups). After just 3 days of incubation, the bands characteristic for the boron-oxygen bond of glasses are invisible in the spectra, which proves their complete transformation into CHA. In case of both glasses modified with ZnO (B40Zn5/s-g/TEB, B40Zn5/s-g/TMBx) after incubation in SBF, the appearance of new bands characteristic for the vibrations of the PO₄³⁻ and CO₃²⁻ groups can also be observed. The broadening of the main band at 1040 cm⁻¹, a significant reduction in the intensity of the bands at 1100 cm⁻¹ and 950 cm⁻¹, the presence of a single band at 560 cm⁻¹, and also a decrease in the intensity of the bands at 870 cm⁻¹ and 1350-1550 cm⁻¹ indicate the presence of an amorphous or very low crystalline calcium-phosphate phase. These changes can also be noticed after 3 days of incubation, in parallel with the complete disappearance of the bands originating from the boron-oxygen bond of glasses. This again proves the complete transformation of glasses into the phosphate-calcium phase, but in case of glasses containing ZnO, having amorphous/lowly crystalline character. Both in case of unmodified and ZnO-modified glasses, a precursor of B₂O₃ used during the synthesis did not have a significant impact on the process of their mineralization in SBF.

Przyk 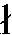 ad 6: *In vitro* bioactivity of some glasses prepared according to examples 1 and 3 - changes in concentration of ions in the incubation fluid.

### Incubation fluid

After incubation of glass powders in artificial plasma, the procedure of which was described in example 5, the concentration of boron, calcium, phosphorus and zinc in the incubation fluid was determined. For this purpose, an ICP-OES Optima 7300 spectrometer (PerkinElmer GmbH, Germany) was used.

In the first three days of incubation, boron and calcium are significantly released from the structure of all glasses, and then their concentration remains stable until the end of the experiment. This confirms the complete transformation of glasses into the calcium-phosphate phase already in the first incubation period. In case of all glasses, a significant decrease in the phosphorus concentration in SBF could be observed in the first three days of incubation. For ZnO-modified glasses (B40Zn5/s-g/TEB, B40Zn5/s-g/TMBx) it is not complete, while in case of unmodified glasses (B40/s-g/TEB, B40/s-g/TMBx) on the third day, phosphorus in the incubation fluid is completely depleted. This corresponds to nature of the calcium phosphate formed as a result of incubation of glasses in SBF - crystalline CHA in case of unmodified glasses and an amorphous phase in case of ZnO-modified glasses. Zinc from the structure of both glasses is gradually released into the SBF over the incubation time. The kinetics of changes in the concentration of calcium, boron and zinc in SBF does not significantly depend on a precursor of B₂O₃ used in the method for producing glass.

In addition to the above-mentioned advantages, as shown in example 4, the method according to the present invention enables the production of glasses with compositions (e.g. glass of the three-component system B₂O₃-CaO-P₂O₅ with a very low content of B₂O₃ and a high content of CaO) that are impossible to obtain using traditional melting techniques, and also very difficult to obtain using known varieties of the sol-gel method. In the proposed solution, it is also possible to use a very wide range of precursors of oxide, which makes this method very flexible, also in terms of the compositions of the obtained glasses.

The invention finds application in various fields of medicine, in treatment of enamel and dentin damage, wound treatment, bone tissue regeneration, soft tissue regeneration, stimulation of angiogenesis; as an antibacterial component of biomaterials; as a component of substrates/scaffolds for tissue engineering; as an ingredient of cosmetics; as a substrate for the production of biomimetic hydroxyapatite; as a component of composite biomaterials. The solution can be used directly in the form of particles (powder), or it can be processed, using processes known in the art, into the form of granules, pastes, porous spatial substrates/scaffolds, coatings/layers on metallic substrates and others.

## Claims

1. A method for producing bioactive borate glasses, **characterized in that** it comprises the following steps:
a) mixing of precursors of B₂O₃, CaO, Na₂O and P₂O₅ in a solvent for 12-48 hours, in a temperature from 20 to 40°C, to obtain a sol;
b) freezing the sol obtained in step a) in a temperature from -10 to -196 °C, and then freeze-drying to obtain an aerogel;
c) grinding the aerogel obtained in step b) and sieving through a sieve with a mesh size of 40-100 µm;
d) heating the aerogel sieved in step c) according to the following temperature treatment scheme:
- heating the aerogel at a speed ranging from 1,5 to 3 °C/min up to a temperature ranging from 100 to 150 °C;
- maintaining the aerogel in a temperature ranging from 100 to 150 °C, over time from 24 to 72 hours;
- heating the aerogel at a speed ranging from 1,5 to 3 °C/min, up to a temperature ranging from 400 to about 600 °C;
- maintaining the aerogel in a temperature ranging from 400 to about 600 °C, over time from 1,5 to 6 hours;
- free cooling of the furnace.

2. The method of claim 1, **characterized in that** the precursor of B₂O₃ is boric acid, trimethyl borate, triethyl borate, tripropyl borate, tributyl borate, tri-tert-butyl borate or trimethoxyboroxin.

3. The method of claims 1-2, **characterized in that** the precursor of CaO is calcium chloride, calcium nitrate, calcium acetate, calcium lactate, calcium citrate, calcium methoxide, calcium ethoxide or calcium methoxyethoxide.

4. The method of claims 1-3, **characterized in that** the precursor of Na₂O is sodium chloride, sodium nitrate, sodium acetate, sodium lactate, sodium citrate, sodium methoxide or sodium ethoxide.

5. The method of claims 1-4, **characterized in that** the precursor of P₂O₅ is trimethyl phosphate, triethyl phosphate, tripropyl phosphate, tributyl phosphate, dibutyl phosphate, phosphoryl trichloride and phosphoric acid.

6. The method of claims 1-5, **characterized in that**, in step a), to the mixture of the precursors of B₂O₃, CaO, Na₂O and P₂O₅ in a solvent, precursors of K₂O, Li₂O, MgO, SrO, TiO₂, MnO₂, Fe₂O₃, CoO, NiO, CuO, ZnO, Ag₂O, Au₂O₃, Al₂O₃ or Ga₂O₃, or combinations thereof, are added.

7. The method of claim 6, **characterized in that** the precursors of K₂O, Li₂O, MgO, SrO, TiO₂, MnO₂, Fe₂O₃, CoO, NiO, CuO, ZnO, Ag₂O, Au₂O₃, Al₂O₃ or Ga₂O₃ are inorganic salts of specific elements, selected from the group comprising chlorides, nitrates, acetates, lactates, citrates and alcoholates.

8. The method of claims 1-7, **characterized in that**, in step a), the solvent is an organic solvent whose melting point is ≥ 0°C.

9. The method of claim 8, **characterized in that** the solvent is 1,4-dioxane, dimethyl carbonate, tert-butanol or an aqueous solution of these compounds.

10. The method of claims 1-9, **characterized in that**, in step a), the volume ratio of the precursor of boron to the solvent is from 1:1 to 1:3.

11. The method of claims 1-10, **characterized in that** the product obtained in step d) is subjected to grinding.

## Patentansprüche

1. Verfahren zur Herstellung bioaktiver Boratgläser, **dadurch gekennzeichnet, dass** es folgende Schritte umfasst:
a) Mischen der Vorläufer von B₂O₃, CaO, Na₂O und P₂O₅ in einem Lösungsmittel für 12 bis 48 Stunden bei einer Temperatur von 20 bis 40 °C, um ein Sol zu erhalten;
b) Einfrieren des in Schritt a) erhaltenen Sols bei einer Temperatur von -10 bis -196 °C und anschließendes Gefriertrocknen, um ein Aerogel zu erhalten;
c) Zerkleinern des in Schritt b) erhaltenen Aerogels und Sieben durch ein Sieb mit einer Maschenweite von 40-100 µm;
d) Erhitzen des in Schritt c) gesiebten Aerogels nach dem folgenden Wärmebehandlungsschema:
- Erhitzen des Aerogels mit einer Geschwindigkeitsrate von 1,5 bis 3 °C/min auf eine Temperatur zwischen 100 und 150 °C;
- Halten des Aerogels in einem Temperaturbereich von 100 bis 150 °C über einen Zeitraum von 24 bis 72 Stunden;
- Erhitzen des Aerogels mit einer Geschwindigkeitsrate von 1,5 bis 3 °C/min auf eine Temperatur zwischen 400 und etwa 600 °C;
- Halten des Aerogels in einem Temperaturbereich von 400 bis etwa 600 °C über einen Zeitraum von 1,5 bis 6 Stunden;
- Abkühlen des Ofens auf natürliche Weise.

2. Verfahren nach Anspruch 1, **dadurch gekennzeichnet, dass** es sich bei dem Vorläufer von B₂O₃ um Borsäure, Trimethylborat, Triethylborat, Tripropylborat, Tributylborat, Tri-tert-butylborat oder Trimethoxyboroxin handelt.

3. Verfahren nach den Ansprüchen 1-2, **dadurch gekennzeichnet, dass** es sich bei dem Vorläufer von CaO um Calciumchlorid, Calciumnitrat, Calciumacetat, Calciumlactat, Calciumcitrat, Calciummethanolat, Calciumethanolat oder Calciummethoxyethanolat handelt.

4. Verfahren nach den Ansprüchen 1-3, **dadurch gekennzeichnet, dass** es sich bei dem Vorläufer von Na₂O um Natriumchlorid, Natriumnitrat, Natriumacetat, Natriumlactat, Natriumcitrat, Natriummethanolat oder Natriumethanolat handelt.

5. Verfahren nach den Ansprüchen 1 bis 4, **dadurch gekennzeichnet, dass** es sich bei dem Vorläufer von P₂O₅ um Trimethylphosphat, Triethylphosphat, Tripropylphosphat, Tributylphosphat, Dibutylphosphat, Phosphoryltrichlorid und Phosphorsäure handelt.

6. Verfahren nach den Ansprüchen 1 bis 5, **dadurch gekennzeichnet, dass** in Schritt a) dem Gemisch der Vorläufer von B₂O₃, CaO, Na₂O und P₂O₅ in einem Lösungsmittel die Vorläufer von K₂O, Li₂O, MgO, SrO, TiO₂, MnO₂, Fe₂O₃, CoO, NiO, CuO, ZnO, Ag₂O, Au₂O₃, Al₂O₃ oder Ga₂O₃ oder Kombinationen davon zugegeben werden.

7. Verfahren nach Anspruch 6, **dadurch gekennzeichnet, dass** die Vorläufer von K₂O, Li₂O, MgO, SrO, TiO₂, MnO₂, Fe₂O₃, CoO, NiO, CuO, ZnO, Ag₂O, Au₂O₃, Al₂O₃ oder Ga₂O₃ anorganische Salze bestimmter Elemente sind, ausgewählt aus der Gruppe umfassend Chloride, Nitrate, Acetate, Lactate, Citrate und Alkoholate.

8. Verfahren nach Anspruch 1-7, **dadurch gekennzeichnet, dass** in Schritt a) das Lösungsmittel ein organisches Lösungsmittel ist, dessen Schmelzpunkt ≥ 0 °C beträgt.

9. Verfahren nach Anspruch 8, **dadurch gekennzeichnet, dass** es sich bei dem Lösungsmittel um 1,4-Dioxan, Dimethylcarbonat, tert-Butanol oder eine wässrige Lösung dieser Verbindungen handelt.

10. Verfahren nach Anspruch 1-9, **dadurch gekennzeichnet, dass** in Schritt a) das Volumenverhältnis von Borvorläufer zu Lösungsmittel 1:1 bis 1:3 beträgt.

11. Verfahren nach Anspruch 1-10, **dadurch gekennzeichnet, dass** das in Schritt d) erhaltene Produkt zerkleinert wird.

## Revendications

1. Procédé de fabrication de verres boratés bioactifs, **caractérisé en ce qu'**il comprend les étapes suivantes :
a) le mélange des précurseurs B₂O₃, CaO, Na₂O et P₂O₅ dans un solvant pendant 12 à 48 heures, à une température comprise entre 20 et 40 °C, afin d'obtenir un sol ;
b) la congélation du sol obtenu à l'étape a) à une température comprise entre -10 et -196 °C, puis la lyophilisation afin d'obtenir un aérogel ;
c) le broyage de l'aérogel obtenu à l'étape b) et le tamisage à travers un tamis dont la taille des mailles est comprise entre 40 et 100 µm ;
d) le maintien à température de l'aérogel tamisé à l'étape c) selon le schéma de traitement thermique suivant :
- le chauffage de l'aérogel à une vitesse comprise entre 1,5 et 3 °C/min jusqu'à une température comprise entre 100 et 150 °C ;
- le maintien de l'aérogel à une température comprise entre 100 et 150 °C pendant 24 à 72 heures ;
- le chauffage de l'aérogel à une vitesse comprise entre 1,5 et 3 °C/min jusqu'à une température comprise entre 400 et environ 600 °C ;
- le maintien de l'aérogel à une température comprise entre 400 et environ 600 °C pendant 1,5 à 6 heures ;
- le refroidissement naturel du four.

2. Procédé selon la revendication 1, **caractérisé en ce que** le précurseur de B₂O₃ est l'acide borique, le borate de triméthyle, le borate de triéthyle, le borate de tripropyle, le borate de tributyle, le borate de tri-tert-butyle ou la triméthoxyboroxyne.

3. Procédé selon les revendications 1 à 2, **caractérisé en ce que** le précurseur de CaO est le chlorure de calcium, le nitrate de calcium, l'acétate de calcium, le lactate de calcium, le citrate de calcium, le méthanolate de calcium, l'éthanolate de calcium ou le méthoxyéthanolate de calcium.

4. Procédé selon les revendications 1 à 3, **caractérisé en ce que** le précurseur de Na₂O est le chlorure de sodium, le nitrate de sodium, l'acétate de sodium, le lactate de sodium, le citrate de sodium, le méthanolate de sodium ou l'éthanolate de sodium.

5. Procédé selon les revendications 1 à 4, **caractérisé en ce que** le précurseur de P₂O₅ est le phosphate de triméthyle, le phosphate de triéthyle, le phosphate de tripropyle, le phosphate de tributyle, le phosphate de dibutyle, le trichlorure de phosphoryle et l'acide phosphorique.

6. Procédé selon les revendications 1 à 5, **caractérisé en ce que**, à l'étape a), au mélange de précurseurs B₂O₃, CaO, Na₂O et P₂O₅ dans le solvant sont ajoutés les précurseurs K₂O, Li₂O, MgO, SrO, TiO₂, MnO₂, Fe₂O₃, CoO, NiO, CuO, ZnO, Ag₂O, Au₂O₃, Al₂O₃ ou Ga₂O₃, ou leurs combinaisons.

7. Procédé selon la revendication 6, **caractérisé en ce que** les précurseurs K₂O, Li₂O, MgO, SrO, TiO₂, MnO₂, Fe₂O₃, CoO, NiO, CuO, ZnO, Ag₂O, Au₂O₃, Al₂O₃ ou Ga₂O₃ sont des sels inorganiques d'éléments spécifiques, choisis parmi le groupe comprenant les chlorures, les nitrates, les acétates, les lactates, les citrates et les alcoolates.

8. Procédé selon les revendications 1 à 7, **caractérisé en ce que**, à l'étape a), le solvant est un solvant organique dont la température de fusion est ≥ 0 °C.

9. Procédé selon la revendication 8, **caractérisé en ce que** le solvant est le 1,4-dioxane, le carbonate de diméthyle, le tert-butanol ou une solution aqueuse de ces composés.

10. Procédé selon les revendications 1 à 9, **caractérisé en ce que**, à l'étape a), le rapport volumique entre le précurseur de bore et le solvant est compris entre 1:1 et 1:3.

11. Procédé selon les revendications 1 à 10, **caractérisé en ce que** le produit obtenu à l'étape d) est soumis à un broyage.
